# EUROPEAN PATENT SPECIFICATION

(11) **EP 3 011 022 B1**
(45) Date of publication and mention of the grant of the patent: **07.03.2018**
(21) Application number: 14735877.4
(22) Date of filing: 18.06.2014
(51) Int. Cl.: C12P 17/04, C12N 9/88, C12P 13/00

(54) **HNL MUTANTS AT LOW PH**
HNL-MUTANTEN BEI NIEDRIGEM PH-WERT
MUTANTS DE HNL À PH BAS

(30) Priority: 21.06.2013 CH 11672013; 11.10.2013 CH 17432013
(43) Date of publication of application: 27.04.2016
(73) Proprietor: Dsm Ip Assets B.V., 6411 The Heerlen (NL)
(72) Inventor: CALLANT, Dominique Monique Charles, 4002 Basel (CH); WIERTZ, Roel Wim, 4002 Basel (CH); PETSCHACHER, Barbara, 4002 Basel (CH); GLIEDER, Anton, 4002 Basel (CH); WEINHANDL, Katrin, 4002 Basel (CH); ACHKAR, Jihane, 4002 Basel (CH); WAECHTER, Ralph, 4002 Basel (CH)
(74) Representative: Seibel-Thomsen, Nadja
(86) International application number: PCT/EP2014/062832
(87) International publication number: WO 2014/202671

(56) References cited:
- WO-A1-2008/071695
- US-A1- 2006 105 434
- GLIEDER A ET AL: "Comprehensive step-by-step engineering of an (R)-hydroxynitrile lyase for large-scale asymmetric synthesis", STRUCTURE, CURRENT BIOLOGY LTD., PHILADELPHIA, PA, US, vol. 42, no. 39, 13 October 2003 (2003-10-13), pages 4815-4818, XP002281488, ISSN: 0969-2126

## Description

The present invention relates to newly identified mutants of (R)-selective hydroxynitrile lyase enzyme (R-HNL), in particular variants of *Pa*HNL5 (hydroxynitrile lyase isoenzyme 5 of *Prunus amygdalus*) which are stable at very low pH, i.e. at pH around 2.5 and lower. In particular, the present invention provides nucleic acids encoding said mutant enzymes, recombinant microorganisms and their use for industrial applications.

Pantolactone racemate can be prepared by a chemical reaction from hydroxypivalaldehyde, sodium cyanide, hydrochloric acid and calcium chloride. The manufacture of (*R*)-pantolactone is of big interest for the chemical industry because of its importance in calcium (*R*)-pantothenate and (*R*)-pantothenol synthesis. Both compounds represent vitamin B5 precursors and find wide application in food, cosmetics and pharmaceuticals.

For the industrial production of enantiopure (R)-cyanohydrins, such as (R)-pantolactone, the use of *R*-HNLs isolated from plants of the Rosaceae family, such as e.g. the isoform 5 from *Prunus amygdalus* (*Pa*HNL5), is preferred over a chemical resolution step, wherein the racemate is chemically converted to (S)-or (R)-pantolactone, both from an economical and an ecological aspect.

The establishment of an industrial production process comprising the step of R-HNL-catalyzed asymmetric hydrocyanation reactions is very challenging: first, said asymmetric reaction has to compete with a spontaneous racemic hydrocyanation reaction occurring in the background. This "background reaction" can only be slowed down by lowering the temperatures to around 25 to 15°C and almost suppressed at low pH values, such as e.g. lower than pH 2. Second, the asymmetric hydrocyanation is only possible if the respective substrate is present in the monomeric form. However, hydroxypivalaldehyde forms a stable dimer in water at room temperature and below, which cannot be used as substrate. Only a harsh acidic pH below 2 (such as e.g. around pH 1) seems to favor the monomeric form, which is highly incompatible with biological transformations using standard enzymes. Hydrolysis of dimers is furthermore possible by applying high temperatures such as at least 65°C or high dilution of the substrate in water, which conditions are also not compatible with the HNL-catalyzed asymmetric hydrocyanation reaction. Thus, a "pre-monomerization" step of the substrate in dimer form to produce the active monomer form using high temperature and low pH conditions is necessary prior to addition of the substrate to the enzymatic reaction mixture.

The native R-HNL enzymes which are able to catalyze the stereoselective conversion of hydroxypivalaldehyde to the corresponding (*R*)-cyanohydrin which can be further converted by acid-catalyzed hydrolysis to (R)-pantolactone are known for more than 15 years (see e.g. Effenberger et al., Tetrahedron: Asym. 1995, 6, 271-282). Native (R)-HNL preparations deliver (R)-pantolactone in insufficient yield (84%) and selectivity, i.e. enantiomeric excess (89% ee). Moreover, these enzymes are not stable at low pH and require use of an organic solvent.

With substitution of the amino acid on position 317 in the sequence of the mature *PaHNL5* enzyme leading to the recombinant enzyme *Pa*HNL5_V317A (see WO 2008/071695) conversion of hydroxypivalaldehyde into the corresponding (R)-cyanohydrin is 13-fold faster than using the native enzyme at low pH and in water. However, due to its semi-stability at pH 2.5 for a longer period, it is still not very efficient for an industrial process under the harsh conditions described above.

Thus, there is an ongoing need for R-HNL variants being active and stable at low pH and low temperature in order to perform asymmetric hydrocyanation of a respective substrate such as e.g. hydroxypivalaldehyde to the corresponding (R)-cyanohydrin with avoidance of both background reaction and dimer formation competing with the asymmetric reaction.

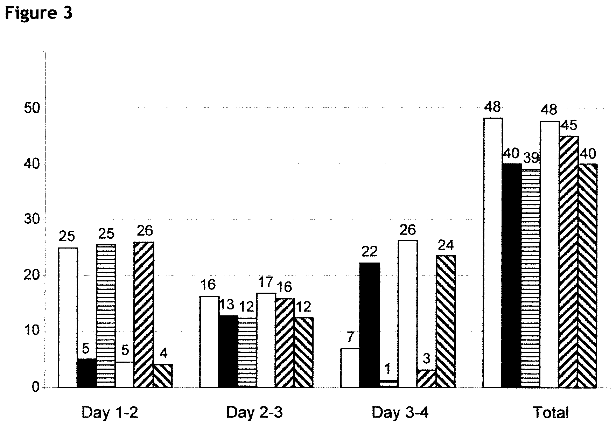

Surprisingly, we now identified amino acid positions which play an important role in stability and activity of R-HNLs under conditions as described above, such as pH around 2.5 and temperatures between 15 to 25°C as required for establishing an industrial process for stereoselective production of (*R*)-pantolactone. Recombinant enzymes have been generated based on the mature PaHNL5 as depicted in SEQ ID NO:1, carrying an amino acid substitution on position 317 and 496, wherein SEQ ID NO:1 corresponds to amino acids 28 to 559 depicted as SEQ ID NO:20 or Figure 3 in EP 1223220, i.e. still including the signal sequence corresponding to the first 27 amino acids in Figure 3 of EP 1223220. Thus, position 317 and 496, respectively, as described in the present application would correspond to position 344 and 523, respectively, as shown in SEQ ID NO:20 of EP 1223220.

Furthermore, a new process has been developed, wherein the stereoselective hydrocyanation is performed at temperatures between around 15°C to 25°C together with pH at around 2.5, in particular a pH cycling between around 2.5 and around 3.0, with possible pH values of 2.6, 2.7, 2.8, 2.9, 3.0. Preferably, these conditions are in combination with a slow substrate addition, i.e. the substrate is continuously or semi-continuously added, i.e. feeding of substrate, such as aldol, to the reaction mixture over at least 3 distinct 1-h long addition periods. Thus, in one embodiment the total amount of substrate is not given within one quick shot but portionwise with e.g. the addition of 30 mmol substrate over 1h in total 3x as described in the Examples. The addition of substrate is correlated with the amount of enzyme added, wherein the substrate is particularly added continuously or semi-continuously at a rate which is proportional to the enzyme used and such that its monomeric form is converted as it is added and does not accumulate. The skilled person will know, in particular looking in the Examples, how to select a feeding rate to avoid spontaneous non-enzymatically catalyzed background reaction. With the novel process, both conversion rates as well as enantiomeric excess could be increased.

In particular, the present invention is directed to a mutant *R*-HNL from Rosaceae family member which is stable at pH 2.5 or lower for at least 1 hour and comprising amino acid substitution(s) on positions corresponding to substitution on a position corresponding to residue 317 as shown in SEQ ID NO:1 which is a substitution of valine by alanine (V317A) and to substitution on a position corresponding to residue 496 as shown in SEQ ID NO:1 which is a substitution of asparagine to serine (N496S).

A recombinant R-HNL derived from *Prunus amygdalus* comprising amino acid substitution, i.e. PaHNL5_V317A, is described in more detail in WO 2008/071695.

Thus, a recombinant R-HNL of the present invention comprises at least a combination of the following amino acid substitutions, i.e. V317A_N496S. Examples of recombinant enzymes according to the present invention originated from *P*. *amygdalus* are selected from *Pa*HNL5_V317A_N496S. Preferably, the R-HNL enzyme used for introducing the herein described amino acid substitutions is derived from *Prunus amygdalus* designated as PaHNL5. The nucleotide sequence of mature *Pa*HNL5 from *Prunus amygdalus* is depicted in SEQ ID NO:1.

The mutant enzyme according to the present invention might carry further mutation(s), including so-called silent mutations. One example of such silent mutation is a nucleotide exchange on a position corresponding to G759A in the respective polynucleotide sequence encoding the enzyme according to SEQ ID NO:1.

The terms "hydroxynitrile lyase", "HNL", "HNL5" or "enzyme" are used interchangeably herein. A hydroxynitrile lyase having the activity to catalyze the stereoselective conversion of a substrate such as e.g. hydroxypivalaldehyde to the corresponding (*R*)-enantiomer as used herein is interchangeably named herein as "R-HNL". The resulting (*R*)-cyanohydrin might be further converted by acid-catalyzed hydrolysis to (R)-pantolactone. The term "PaHNL5" means isoenzyme 5 derived from *Prunus amygdalus.* The amino acid sequence of the mature PaHNL5 is depicted in SEQ ID NO:1. A hydroxynitrile lyase comprising the amino acid substitutions described herein is said to be a "recombinant", "mutant" or "modified" enzyme. The PaHNL5 as well as the mutant enzymes disclosed herein are all able to stereoselectivity convert a substrate such as e.g. hydroxypivalaldehyde to the corresponding (*R*)-enantiomer and thus can be named as R-HNLs.

The recombinant hydroxynitrile lyases described herein can be easily obtained by introducing the one or more mutations in a naturally occurring R-HNL, such as for instance hydroxynitrile lyases of the *Rosaceae* family, in particular enzymes selected from *Prunus amygdalus* (i.e. PaHNL), *Prunus serotina* (i.e. *Ps*HNL), *Prunus avium, Prunus laurocerasus, Prunus lyonii, Prunus armaniaca, Prunus persica, Prunus domestica* (i.e. *Pd*HNL), *Prunus mume* (i.e. *Pm*HNL), *Malus communis, Malus pumila or Cydonia oblonga,* or synthetic *R*-HNLs, examples of which are described in EP 1223220, EP 1566441, WO 2004/083424 or WO 2006/076965. In particular, reference is made to Figure 3 of EP 1223220 showing an amino acids sequence of *Pa*HNL5 including the signal sequence marked in bold. R-HNLs which do not carry one or more of the mutations described herein are said to be "non-modified" enzymes.

Methods for introducing mutations, e.g., additions, deletions and/or substitutions into the nucleotide and/or amino acid sequence of the non-modified R-HNL include but are not limited to site-directed or random mutagenesis and PCR-based methods. The skilled person knows how to introduce mutations, in particular amino acid substitutions into the respective sequence (see e.g. Sambrook et al., Molecular Cloning, Cold Spring Harbor Laboratory Press, New York). A particular useful method is the so-called error prone polymerase chain reaction (ep-PCR). This involves using either DNA polymerases with a high intrinsic error rate, such as, for example, Mutazyme® I DNA polymerase or Mutazyme® II DNA polymerase from Stratagene (CA, USA), or designing the conditions of said polymerase chain reaction in such a way that the error rate increases. The addition of divalent cations, a nucleotide imbalance, pH alteration or a combination of two or three of these options, result in suitable alterations in the PCR reaction mixture for DNA polymerases, such as, for example, *Taq* DNA polymerases from Qiagen, Fermentas, etc. or Hot Start*Taq* DNA polymerases from Fermentas or HotStarTaq DNA polymerases from Qiagen, etc., to incorporate errors with a controllable rate during amplification of the gene. This method is described in more detail in WO 2008/071695.

The libraries obtained by ep-PCR can be screened for increased stability at low pH based on e.g. an activity assay using mandelonitrile as substrate, wherein R-HNL enzyme activity is measured by taking advantage of the formation of UV-active benzaldehyde upon HNL-catalyzed decomposition of racemic mandelonitrile. Absorption measurement can be performed at 280 nm (extinction coefficient for benzaldehyde ε = 1.376 L mmol⁻¹ cm⁻¹) for 2 to 5 min. 1 unit (AMU) corresponds to the amount of HNL enzyme that converts 1 µmοl mandelonitrile into benzaldehyde and HCN per minute at pH 5.0 and room temperature. The skilled person is aware of such and further methods to measure the enzyme activity.

Stereoselective conversion of hydroxypivalaldehyde to the corresponding (*R*)-cyanohydrin can be monitored via gas chromatography, wherein samples are withdrawn at regular time intervals (e.g. t = 5 min, 10 min, 30 min, 1 h, 2 h, 4 h, and 20 h) throughout the course of the reaction. Reaction aliquots are dried by quick addition to an ice-cold suspension of Na₂SO₄ in dichloromethane and stirred for 15 min in an ice-water bath. The mixture is then acetylated by treatment with AC₂O and pyridine including a C15 alkane as internal standard. The mixture is further processed via suspension/decantation, filtered and concentrated in vacuo at 20°C. Product yields are determined by gas chromatography based on response factors established for authentic samples relative to pentadecane as the internal standard (ISTD) after derivatization with acetic anhydride in pyridine. Each GC run lasts 53.5 min and retention times are measured for aldol-monomer (3.99 min), R-cyanohydrin (14.57 min), S-cyanohydrin (14.93 min), glycerol (16.52 min), ISTD (20.85 min), diastereomeric dimer educts I R/S (31.96 min), II R/S (32.62 min), II S/R (32.88 min) and I S/R (33.15 min). This is only one example of a suitable method, further methods are known in the art or described in more detail in the examples.

Expression of recombinant enzymes is carried out in heterologous or secretory expression systems, with preferred host (micro)organisms being selected from *Pichia pastoris, Saccharomyces cerevisiae, Kluyveromyces lactis, Aspergillus niger, Penicillium chrysogenum, Pichia methanolica, Pichia polymorpha, Hansenula polymorpha, Pichia anomala, Schizosaccharomyces pombe, Arxula adeninivorans, Yarrowia lipolytica, Bacillus subtilis, Pseudomonas fluorescens, Escherichia coli, Bacillus stearothermophilus, Thermus thermophilus,* or *Streptomyces* sp., particularly *Escherichia coli, Saccharomyces cerevisiae, Kluyveromyces lactis, Aspergillus niger* or *Pichia pastoris,* with *Aspergillus niger* or *Pichia pastoris* being more preferred, such as e.g. variants of P. pastoris GS115 including *Pichia pastoris* GS115 strains CBS7435 mut^{S} pPpT4 (Näätsaari et al., PLOS one, Vol. 7, issue 6, e39720, 2012).

For expression of the recombinant enzymes in the respective host, the natural or plant signal sequence can be replaced with another signal sequence, for example with the signal sequence of the *Saccharomyces cerevisiae* alpha mating factor (Alpha-MF), *Saccharomyces cerevisiae* invertase (SUC2), the *Pichia* killer toxin signal sequence, *a-amylase, Pichia pastoris* acid phosphatase (PHO1), *Phaseolus vulgaris* agglutinin (PHA-E); the *Aspergillus niger* glucoamylase signal sequence (glaA), the *Aspergillus niger* glucose oxidase signal sequence (GOX), the *Pichia pastoris* Sec10 signal sequence, the signal sequence of the 28 kD subunit of the *Kluyveromyces lactis* killer toxin, the BSA signal sequence, or synthetic variants thereof, or else with synthetic consensus sequences. The signal sequences may also comprise point mutations, insertions or deletions.

Cultivation of the host can be done in batch, fed-batch, continuous, or semi-continuous mode. The skilled person knows how to express a heterologous enzyme in a host system as described herein, in particular using *Pichia* or *Aspergillus* as host organism, in particular *Pichia pastoris* GS115 strains. The recombinant enzyme might be expressed in its native form or fused to an active or inactive tag, such as e.g. glucoamylase (GLA) tag. A sample protocol of an expression of recombinant enzymes defined herein is given as follows: two 20-l fermentations are carried out as fed-batch for each strain and the fermentation broths are pooled together for down-stream processing. The specific activities of the mutant enzymes are evaluated by SDS-PAGE. Fermentation starts with 2 seed steps, with a first seed cultivation for 24 h followed by a second seed step for 8 h. For the subsequent main fermentation, a batch phase of up to 16 hour is followed by production of biomass for another 6 hours or less, starting with a first feeding of glycerol. After a second feed of methanol the actual enzyme production is initiated. Fermentations are terminated after a maximum of 112 hours. Downstream processing encompasses removal of biomass by centrifugation, concentration via ultrafiltration and furthermore filtration which finally results in an R-HNL recovery yield of 90%.

All microorganisms which can be used for the present invention may be publicly available from different sources, e.g., Deutsche Sammlung von Mikroorganismen und Zellkulturen (DSMZ), Inhoffenstr. 7B, D-38124 Braunschweig, Germany, American Type Culture Collection (ATCC), P.O. Box 1549, Manassas, VA 20108 USA or Culture Collection Division, NITE Biological Resource Center, 2-5-8, Kazusakamatari, Kisarazu-shi, Chiba, 292-0818, Japan (formerly: Institute for Fermentation, Osaka (IFO), 17-85, Juso-honmachi 2-chome,Yodogawa-ku, Osaka 532-8686, Japan).

In connection with the present invention it is understood that the above-mentioned microorganisms also include synonyms or basonyms of such species having the same physiological properties, as defined by the International Code of Nomenclature of Prokaryotes. The nomenclature of the microorganisms as used herein is the one officially accepted (at the filing date of the priority application) by the International Committee on Systematics of Prokaryotes and the Bacteriology and Applied Microbiology Division of the International Union of Microbiological Societies, and published by its official publication vehicle International Journal of Systematic and Evolutionary Microbiology (IJSEM).

In one embodiment, the present invention provides a novel process for asymmetric hydrocyanation using low temperature which is around 15°C, such as a temperature in the range of a maximum of 25°C and a minimum of 15°C, such as 25°C, 20°C, 17°C or 15°C and a pH which is around 2.5, with a maximum pH of 3.0 and a minimal pH of 2.5, such as e.g. in the range of 3.0 to 2.5, in particular pH 3.0, 2.5 combined with increased activity and stability of the used hydroxynitrile lyase enzymes. In particularly useful are the recombinant enzymes as defined herein.

A cycling of pH is preferred, i.e. starting the reactions at pH around 2.5 with a shift to a pH around 3.0 after e.g. 24 hours with running the reactions for another 24 hours or more at pH around 3.0, or starting with a pH of 2.5 and a shift to pH 3.0 after 8 h with running the reaction for another 16 hours at pH 3.0. Using such setup, a yield of around 90% and more with a conversion rate of at least around 97% and an enantiomeric excess of at least around 85% when using 3 kAMU of recombinant enzyme can be achieved.

In a preferred embodiment, the stereoselective hydrocyanation reactions using a recombinant enzyme as described herein is performed at a pH between 3.0 and, at a temperature between 25°C and 15°C, such as 25, 20, 17, 15°C. More preferred is a reaction at a pH range of 3.0 to 2.5 and a temperature of 15°C.

Normally, the stereoselective hydrocyanation reactions are performed over 7 days, but also shorter periods are suitable, i.e. 3 or 4 days. Using the mutant enzymes as described herein, a 7 day reaction at pH 3.0 at 15°C yields in up to 93% yield, with a conversion of more than 99%, a selectivity of up to 100% with an enantiomeric excess (ee) of around 76-80%. If the pH is lowered to 2.5, an ee of around 46-55% is achieved after a 4 days reaction. Under the same conditions but with only 2 day reaction, the ee is around 83-85% with 100% conversion. An ee of around 92% obtained with pH 2.5 at 15°C for 2 days dropped to 68% after 4 days, wherein the temperature was fixed at 15°C for 8 hours followed by 12 hours at 25°C on day 3-4 of the reaction (see Table 6).

In one particular embodiment, stability and activity of the recombinant enzymes as defined herein was evaluated by the following experimental set-up composed of three separate 20-hour reactions carried out successively with the same enzyme. The first of these 20-hours reactions is intended to produce enough (R)-cyanohydrin. The second 20-hours reaction is aimed at actually evaluating enzyme performance under process-relevant conditions. The third 20-hours reaction is intended to evaluate enzyme stability. It can be started either directly 4 h after the previous one was stopped or it can be set up after stirring the enzyme under the reaction conditions, meaning in presence of 20 wt% product concentration, excess HCN, and under harsh pH and temperature conditions, for up to 72 h. For the 3 distinct 20-hour reactions liquid enzyme (1 or 3 kAMU) is charged in the reactor once at the onset of the reactions series under controlled pH and temperature conditions, then monomerized aldol (3.05 g) is slowly added to the reaction mixture via a heated pump (80°C, over 1h or 6h, and at a rate of 0.5 g aldol/h) and HCN (1.5 mol. eq.) is added portionwise.

At the end of each reaction, excess HCN is removed by stripping with nitrogen gas until no more HCN is detected in the off-gas using a Dräger single-gas monitor. Product concentration is maintained at 20 wt% starting from the end of Day 1 onwards. The same enzyme is reused twice more to evaluate performance and stability: either on Day 2 and again 24 h later on Day 3 or on Day 2 and again on Day 6 after stirring for 72 h under controlled reaction conditions. Beyond the usual conversion, yield, and enantiomeric excess determinations, the relative contributions of enzyme-catalyzed hydrocyanation versus competing background reaction to the formation of (R)-cyanohydrin are calculated. The spontaneous background reaction leading to racemic (R,S)-cyanohydrin can be calculated based on detected (S)-cyanohydrin and equals to twice the amount of detected (S)-isomer. (R)-cyanohydrin produced from the enzyme-catalyzed reaction alone can be calculated by subtracting the amount of detected (S)-isomer to the total amount of detected (R)-isomer.

For the stereoselective hydrocyanation reaction substrates selected from aliphatic, aromatic or heteroaromatic aldehydes and ketones are mixed with a cyanide group donor in the presence of the recombinant enzymes and under conditions as defined herein. The substrate might be directly added to the reaction mixture, i.e. without any further purification, or in crystallized form.

Preferred aliphatic aldehydes are straight-chain or branched aldehydes having in particular from 2 to 30 carbon atoms, preferably from 4 to 18 carbon atoms, which are saturated or mono- or polyunsaturated in from 1 to 6 positions. Said aldehyde may have both C-C double bonds and C-C triple bonds. Furthermore, the aliphatic, aromatic or heteroaromatic aldehydes may be unsubstituted or by optionally substituted aryl or heteroaryl groups such as phenyl, phenoxy or indolyl groups, by halogen, hydroxy, hydroxy C₁-C₅ alkyl, C₁-C₅ alkoxy, C₁-C₅ alkylthio, ether, alcohol, carboxylate, nitro or azido groups. The term C₁-C₅ refers to the number of C-atoms, including 1, 2, 3, 4, and 5 C-atoms. Particularly preferred are selected from β-hydroxyaldehydes, including but not limited to 3-hydroxy-2,2-dimethyl-propanal (hydroxypivalaldehyde), pivaldehyde, propionaldehyde, butyraldehyde, hexanal, isobutyraldehyde, 3-methylbutanal (isovaleraldehyde), propenal (acrolein), pentanal (valeraldehyde), crotonaldehyde, 2-methylacrolein (methacrolein), 3-phenylpropanal, 3-phenylpropenal (cinnamaldehyde), 3-phenylpropynal, 3-hydroxybutanal (aldol). Further examples of suitable substrates are listed in WO 2008/071695. In a typical experimental setup on a laboratory scale, 3 g of monomerized aldol is slowly added to the reaction, i.e. addition via a heated pump at 80°C for 1 hour, with repetition twice at 2 hours intervals, so that a total amount of 9.16 g (90 mmol) of monomerized aldol is added to a 1 day reaction. For reactions longer than 1 day, this procedure is repeated again with addition of monomerized aldol (3 x 30 mmol, 3 x 3.05 g, over 3 x 1 h) via a heated pump system (for more details see Examples). It turned out that addition of monomerized aldol in one quick shot resulted in good conversion (88%) but lower ee (78%). Thus, a slow addition of monomerized aldol in 1 h or 2 h with high conversions (95%) and high ee (88%) is preferred, such as e.g. addition of monomerized aldol (e.g. 3.05 g, 30 mmol) to the reaction mixture via a heated pump at 80°C for 1h or 6h, at a rate of 0.5 g aldol/h.

Suitable cyanide group donors are hydrocyanic acid, alkaline metal cyanides or a cyanohydrin of the general formula

R₁R₂C(OH)(CN)

wherein R₁ and R₂ independently of one another are hydrogen or an unsubstituted hydrocarbon group, or R₁ and R₂ are together an alkylene group having 4 or 5 carbon atoms, with R₁ and R₂ not being hydrogen at the same time. The hydrocarbon groups are aliphatic or aromatic, preferably aliphatic, groups. R₁ and R₂ are preferably alkyl groups having from 1-6 carbon atoms, with more preference to hydrocyanic acid (HCN), KCN, NaCN, or acetone cyanohydrin, most preferably HCN. HCN can be generated from hydrogen cyanide in situ from sodium cyanide and sulfuric acid. The resulting crude HCN solution is either used directly or HCN is further purified by distillation and added in its pure liquid form. The skilled person well knows methods to produce a cyanide group donor such as HCN or other suitable compounds.

Amounts of added HCN ranged from 1.0 to 6.0 molar equivalents relative to aldol. A limitation in HCN can be observed at higher enzyme loadings. Under conditions where HCN is limiting (1.5 mol eq. HCN and 500 - 4000 U HCN/g aldol) or at lower enzyme loadings (500 U HNL/g aldol and up to 4.5 mol eq. HCN), unreacted monomer reverts to dimer, the enzyme-catalyzed reaction is slower but the background reaction suppressed resulting in a final enantiomeric excess over 92%. When HCN is in excess (4.5 mol eq.), it is important to increase the amount of R-HNL (> 2000 U/g aldol) in order to decrease the speed of the background reaction leading to low final enantiomeric excess. When both HCN (6 mol eq.) and enzyme loading (8000 U/g aldol) are high the amount of monomer becomes limiting. With HCN in excess (4.5 mol eq.) and high enzyme loadings (>2000 U/g) the enzyme reaction can be accelerated with an overall fast and high conversion and high enantiomeric excess. In a particular embodiment, the amount of HCN is set between 1.5 to 4.5 molar equivalents relative to aldol under conditions of fast (10 min) addition of monomerized aldol, with a good enantiomeric excess and high enzyme loading. Thus, a low but not limiting HCN concentration is preferred which is added slowly, i.e. portion wise. In particular, amounts of 1.2-1.5 molar equivalents relative to aldol added in 6 equal portions with 0.25 molar equivalents per portion or added in a portion of 0.3 molar equivalents at the onset of aldol addition followed by 0.9 molar equivalents per portion, are preferred. In another embodiment, HCN is added in 3 portions of 1.2 molar equivalents relative to aldol.

The stereoselective hydrocyanation reactions described above are run as batch, continuous or semi-continuous process, wherein semi-continuous mode is preferred. When using a semi-continuous process, slow feeding rates as described above for both the substrate and the cyanide donor, such as e.g. HCN, are preferred. The amount of recombinant enzyme is not critical for the above-described hydrocyanation reaction. Preferred are concentrations in the range of 1-9 kAMU, in particular 3-9 kAMU, such as e.g. 1, 2, 3, 6 or 9 kAMU wherein 1 unit (AMU) corresponds to the amount of HNL enzyme that converts 1 µmοl mandelonitrile into benzaldehyde and HCN per minute at pH 5.0 and room temperature.

The reaction might be performed in the presence of further stabilizing compounds, such as e.g. FAD, as known in the art.

This invention is further illustrated by the following examples which should not be construed as limiting. Reference is made in particular to EP 1223220, WO 01/21772, WO 02/057474, WO 02/061108, WO 2004/113510A2 or WO 2007/065602A1.

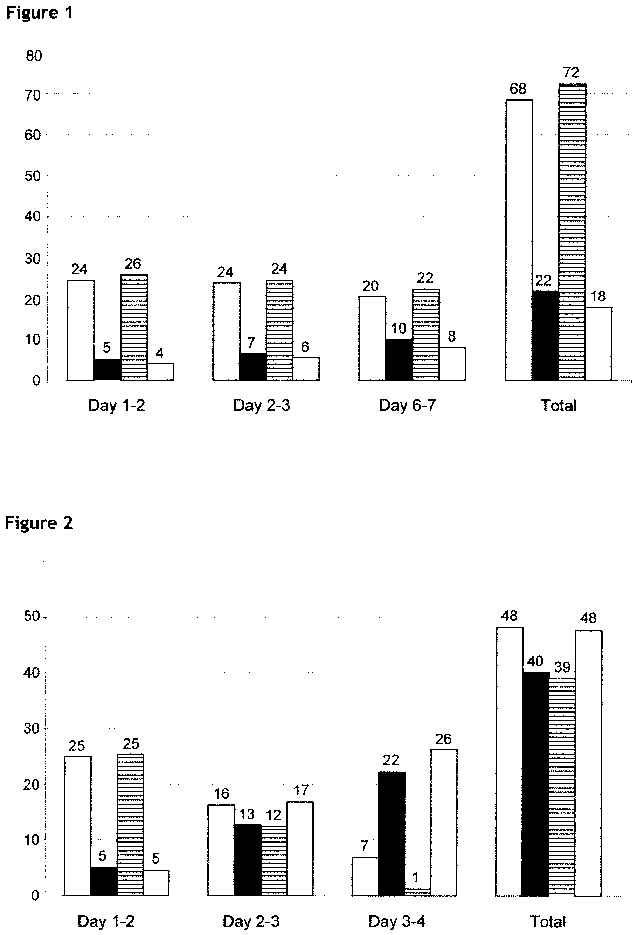

Figure 1: Comparison of PaHNL5_V317A_N496A and PaHNL5_V317A_N496S at pH 3.0 and 15°C in terms of enzyme-catalyzed versus background reaction. The x-axis shows the time, the y-axis shows the formed product in mmol. Enzyme reaction for PaHNL5_V317A_N496S is depicted as white column, background reaction for PaHNL5_V317A_N496S is depicted as black column. For PaHNL5_V317A_N496A, enzyme reaction is shown as column with horizontal stripes, background reaction is shown as grey column.
Figure 2: Comparison of PaHNL5_V317A_N496A and PaHNL5_V317A_N496S at pH 2.5 and 15°C in terms of enzyme-catalyzed versus background reaction. The x-axis shows the time, the y-axis shows the formed product in mmol. For color code, see legend to Figure 1.
Figure 3: Comparison of PaHNL5_V317A_N496A, PaHNL5_V317A_N496S and PaHNL5_V317A at pH 2.5 and 15°C in terms of enzyme-catalyzed versus background reaction. The x-axis shows the time, the y-axis shows the formed product in mmol. Enzyme reaction for PaHNL5_V317A is depicted as white upward diagonal stripped column, background reaction for PaHNL5_V317A is depicted as white downward diagonal stripped column. For further color code, see legend to Figure 1.

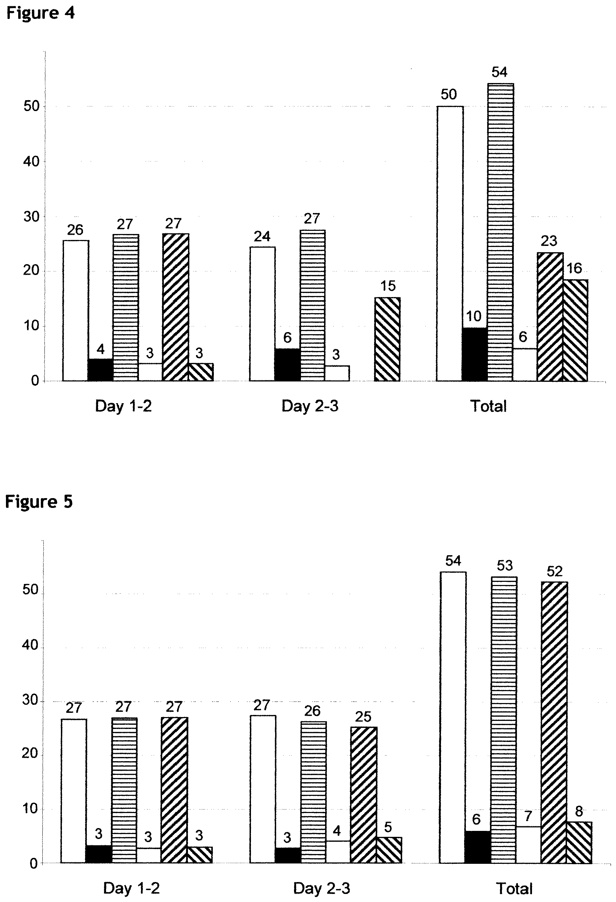

Figure 4: Performance of PaHNL5_V317A_N496S under different pH conditions (see Table 4). From Day 2 on, the product concentration was around 20% and aldol was fed in 1 h at different pH-levels: Enzyme and background reaction, respectively, are shown as white and black columns for pH 3.0, as horizontal stripped or grey columns for pH 2.5 and as white upward diagonal stripped or white downward diagonal stripped columns for pH 2.0. For more explanation see text.
Figure 5: Performance of PaHNL5_V317A_N496S under different temperature conditions with fixed pH at 2.5 (see Table 5). From Day 2 on, the product concentration was around 20% and aldol was fed in 1 h at different temperatures: Enzyme and background reaction, respectively, are shown as white and black columns for 15°C, as upwards horizontal stripped or grey columns for 20°C and as white upward diagonal or white downward diagonal stripped columns for 25°C. For more explanation see text.

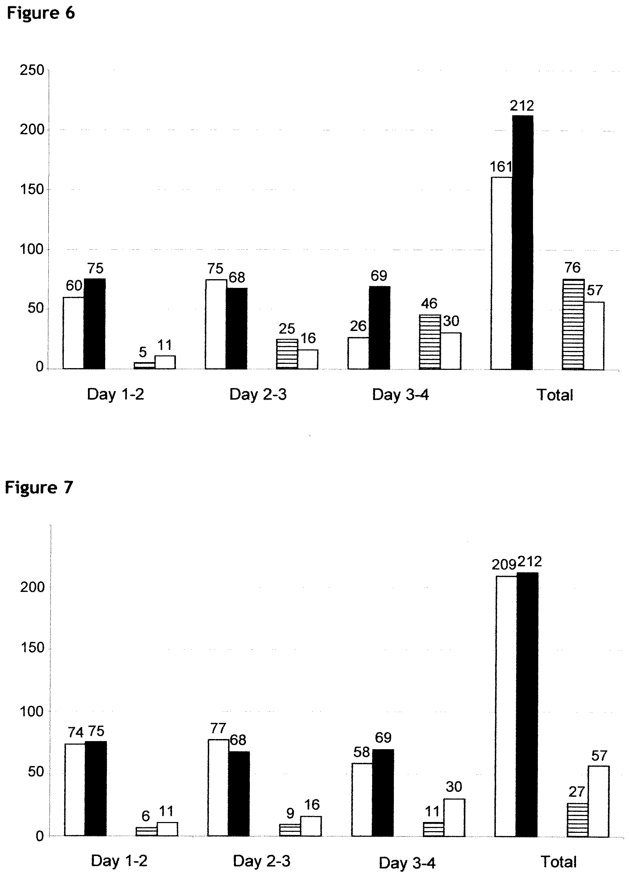

Figure 6: Performance of PaHNL5_V317A_N496S under standard enzyme loading (3 kAMU) at pH 2.5 (white and upwards horizontal stripped columns) or pH 3.0 (black and grey columns) at 15°C (see Table 6). The enzyme reaction is shown in the first, third, fifths, seventh pair of columns, background reaction is shown in the second, forth, sixth, and eighths pair of columns. The experiments were performed under fed-batch conditions with semi-continuous substrate addition. For more explanation see text.
Figure 7: Performance of PaHNL5_V317A_N496S under standard (9 kAMU: white and upwards horizontal stripped columns) and increased (3 kAMU: black and grey columns) enzyme loading at pH 3.0 and 15°C in terms of enzyme reactions shown in the first, third, fifths, and seventh pair of columns, and background reactions shown in the second, forth, sixth, and eighths pair of columns. The experiments were performed under fed-batch conditions with semi-continuous substrate addition. For more explanation see text.

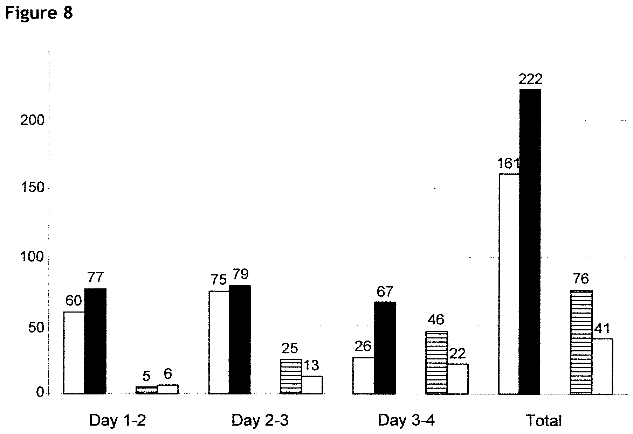

Figure 8: Performance of PaHNL5_V317A_N496S under standard (3 kAMU) enzyme loading at 15°C and with pH fixed at 2.5 (white and upwards horizontal columns) or cycling between 2.5 for 8 h and 3.0 for 16 h (black and grey columns) in terms of enzyme and background reactions under fed-batch conditions with semi-continuous substrate addition. The enzyme reaction is shown in the first, third, fifths, seventh pair of columns, background reaction is shown in the second, forth, sixth, and eighths pair of columns. For more explanation see text.

### Examples

### Example 1: Generation and screening of mutant enzymes for increased stability at low pH

Modification was done by error-prone (ep)-PCR, whereby an imbalanced dNTP mixture (higher dCTP/dTTP concentration, 2mM) and additional 2 mM MgCl₂ together with Taq-Polymerase was used. The template DNA was a pJET-vector comprising the PaHNL_V317A gene (originally obtained from *Prunus amygdalus*) and a linker for enabling overlap extension (oe) PCR (for method see WO 2008/071695, in particular Examples 1 and 2).

The resulting expression cassettes constructed by oe-PCR contained a promoter (e.g. GAP or AOX1), the *S*. *cerevisiae* alpha-factor leader sequence, the PaHNL_V317A gene mutated by ep-PCR, a terminator and a Zeocine-resistance cassette. The whole expression cassette was transformed into *Pichia pastoris* CBS7435 mut^{S} using the "Condensed protocol" (Lin-Cereghino et al., Biotechniques. 2005; 38 (1):44, 46, 48). Transformants were picked and cultivated in deep well plates for 120 h in BMD medium with 7.5 g/l glucose without induction (for protocol, see Example 3 of WO 2008/071695).

From the library constructed by ep-PCR, double mutant V317A_N496S was isolated for its improved stability at low pH. The sequence is depiced in SEQ ID NO:2. Besides V317A_N496S, two further variants were constructed, i.e. V317A_N496A and V317A_N496D, and tested for their activity at low pH. Mutant enzymes were named R-HNL5_V317A_N496S, R-HNL5_V317A_N496A, and R-HNL5_V317A_N496D, respectively.

### Example 2: Production and recovery of mutant enzymes by fermentation

Double-mutant enzymes PaHNL5_V317A_N496S, PaHNL5_V317A_N496A and PaHNL5_V317A_N496S as well as PaHNL5_V317A were produced by fermentation from the corresponding recombinant *Pichia pastoris* GS115 strains CBS7435 mut^{S} pPpT4 (Näätsaari et al., PLOS one, Vol. 7, issue 6, e39720, 2012). Two 20-L fermentations were carried out for each strain and the fermentation broths were pooled together for down-stream processing according to the protocol disclosed in Example 12 of WO 2008/071695. In brief, a 1^{st} seed of 150 ml inoculated with a single colony of the corresponding recombinant *Pichia pastoris* GS115 strain was cultivated for 24 h, followed by a 2^{nd} seed for 8 h in 600 ml medium. Subsequent main fermentation was performed in 3 steps with the output of the 2^{nd} seed as inoculum. After 16 hours of batch phase, a first glycerol feed was added and the cultivation was continued for another 6 h to allow biomass production. Production of recombinant enzymes was initiated by feeding methanol - which served as inducer - with cultivation for another 89 h. Harvesting and further concentration of the enzymes was done as described in WO 2008/071695. After a total time of 112 hours, the fermentation broth containing the recombinant enzyme was centrifuged in order to remove biomass. The supernatant was concentrated by ultrafiltration and further filtered to reduce germs. An enzyme recovery yield of approximately 90% could be achieved.

The specific activities of the double-mutant enzymes were evaluated by SDS-PAGE, whereby samples were diluted to the same activity concentration (10 AMU/g) before sample treatment and loading of 0.065 AMU per lane onto the gel. Gels were stained with Sypro®Ruby then scanned on a Typhoon Scanner. Relative protein quantification was carried out using ImageQuant™ TL software and reported using PaHNL5_V317A as reference.

### Example 3: Performance of PaHNL5 mutants at pH 3.0 and 15°C

For testing the performance of the variants at pH 3.0 and 15°C, two series of experiments were carried out under the same reaction conditions using either mutant enzyme PaHNL5_V317A_N496A or mutant enzyme PaHNL5_V317A_N496S. These experiments spanned over seven days and each involved three sequential 20-hours reactions wherein liquid enzyme from the same enzyme batch was charged in the reactor once at the onset of the reaction series under controlled pH and temperature conditions. On Day 1, 1000 AMU (330 AMU/g aldol; 2000 AMU/g/h aldol dosage) of the appropriate enzyme was charged into the reactor while controlling the pH at 3.0 and temperature at 15°C. Aldol substrate (30 mmol, 3.05 g), which had been purified by crystallization, taken up in water to form a 20% aqueous solution then monomerized at 75°C for 2-3 h, was slowly added on Day 1, on Day 2, and again on Day 6 over 6 h (rate of 0.5 g aldol/h) via a heated pump set to 80°C. In parallel on Day 1, on Day 2, and again on Day 6, freshly prepared HCN (1.5 mol eq. relative to aldol) was added in 6 equal portions (0.25 mol eq. per portion via a custom-made precision addition funnel) every hour during aldol addition. At the end of each reaction, excess HCN was removed by stripping with nitrogen gas until no more HCN was detected in the off-gas using a Dräger single-gas monitor. From Day 2 onwards, product concentration in the reaction mixture was steady at 20 wt%. Reactions were monitored by gas chromatography using standard sample preparation and analytical methods. Between Day 3 (end of the second reaction) and Day 6 (onset of the third reaction), the enzymes were stirred at pH 3.0 and 15°C while exposed to residual HCN and 20 wt% (R)- and (S)-cyanohydrin in the reaction mixture. Using this semi-continuous system, enzymes could be compared in terms of overall performance (Table 1A and 1B) and in terms of stability by observing which double-mutant enzyme led to a lesser background reaction (Figure 1).

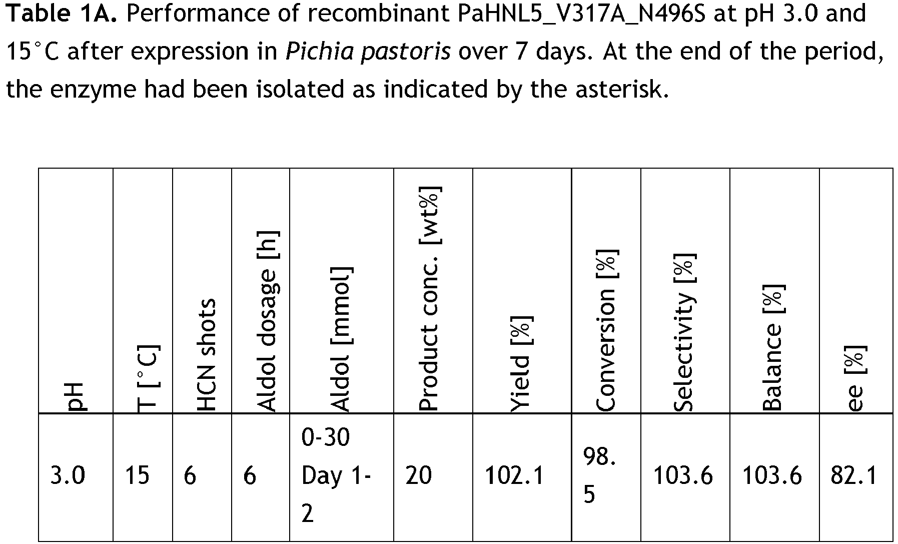

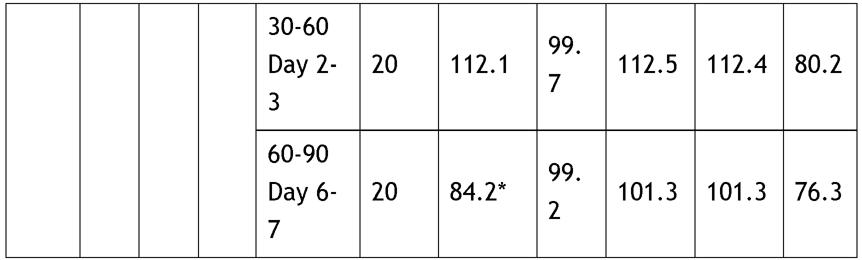

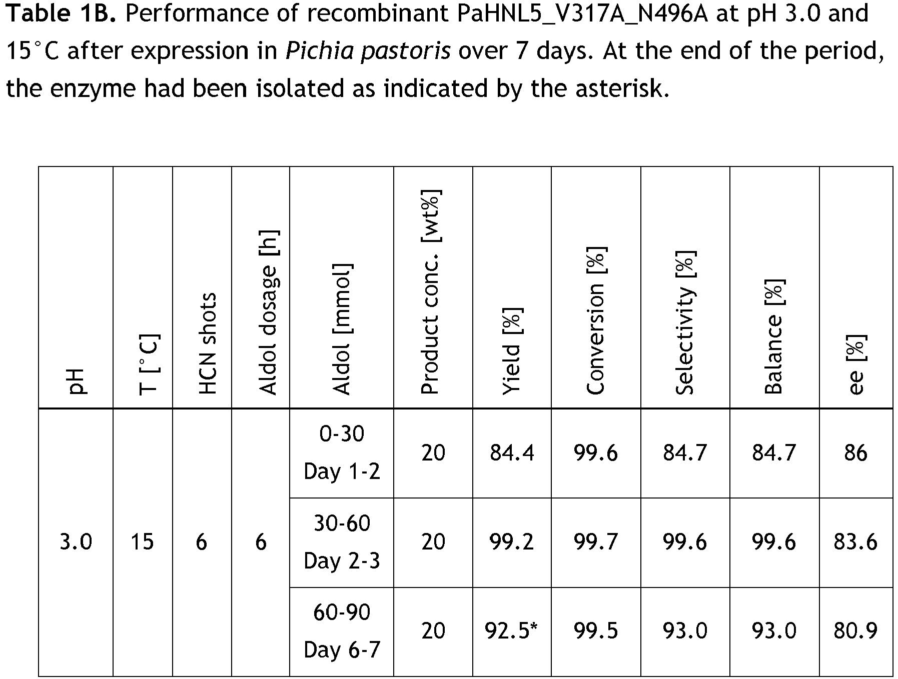

PaHNL5_V317A_N496A seemed slightly more stable than PaHNL5_V317A_N496S as judged by the better corresponding final enantiomeric excess (ee of 81% versus 76%, respectively) recorded at the end of the seven-day period (Table 1) and by the lower competition from the background reaction, more particularly on Day 6 (Figure 1).

### Example 4: Performance of PaHNL5 mutants at pH 2.5 and 15°C

Experiments were carried out with both PaHNL5_V317A_N496S and PaHNL5_V317A_N496A at pH 2.5 and 15°C in order to further investigate activity and stability of the mutant enzymes. The experiment was run for 4 days and involved 3 sequential reactions using the same enzyme batch. On Day 1, 1000 AMU (330 AMU/g aldol; 2000 AMU/g/h aldol dosage) of the appropriate enzyme was charged into the reactor. Crystallized aldol was monomerized at 75°C for 2-3 h and added as a 20% aqueous solution over 6 h via a heated pump set to 80°C. On Day 1, on Day 2, and again on Day 3, monomerized aldol (30 mmol, 3.05 g) was slowly added to the reaction mixture. In parallel on Day 1, on Day 2, and again on Day 3, freshly prepared HCN (1.5 mol eq. relative to aldol) was added in 6 equal portions (0.25 mol eq. per portion) every hour during aldol addition. From Day 2 onwards, product concentration in the reaction mixture was steady at 20 wt%. The pH-stability of double mutants PaHNL5_V317A_N496A and PaHNL5_V317A_N496S was furthermore compared to stability of PaHNL5_V317A expressed from *Aspergillus niger.* Reactions were monitored by gas chromatography as described above.

None of the enzymes could withstand a low pH of 2.5 for more than 1 day. Interestingly, PaHNL5_V317A_N496S seems slightly more stable than PaHNL5_V317A which is in turn more stable than PaHNL5_V317A_N496A as judged by the recorded final ee of 46% versus 55%, respectively (Table 2A and 2B) and by the higher background reaction in presence of PaHNL5_V317A_N496A (Figure 2). Also, PaHNL5_V317A_N496S was the only enzyme to display some minute catalytic activity on Day 3 at pH 2.5. It seems that after 96 h PaHNL5_V317A_N496A lost all catalytic activity whereas some residual enzymatic activity could still be measured for PaHNL5_V317A_N496S.

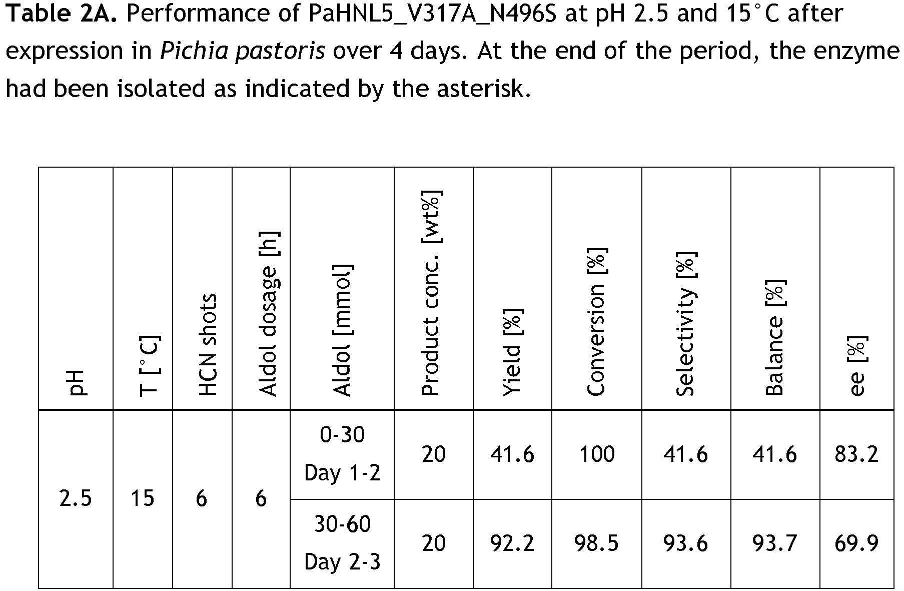

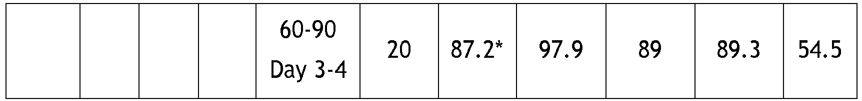

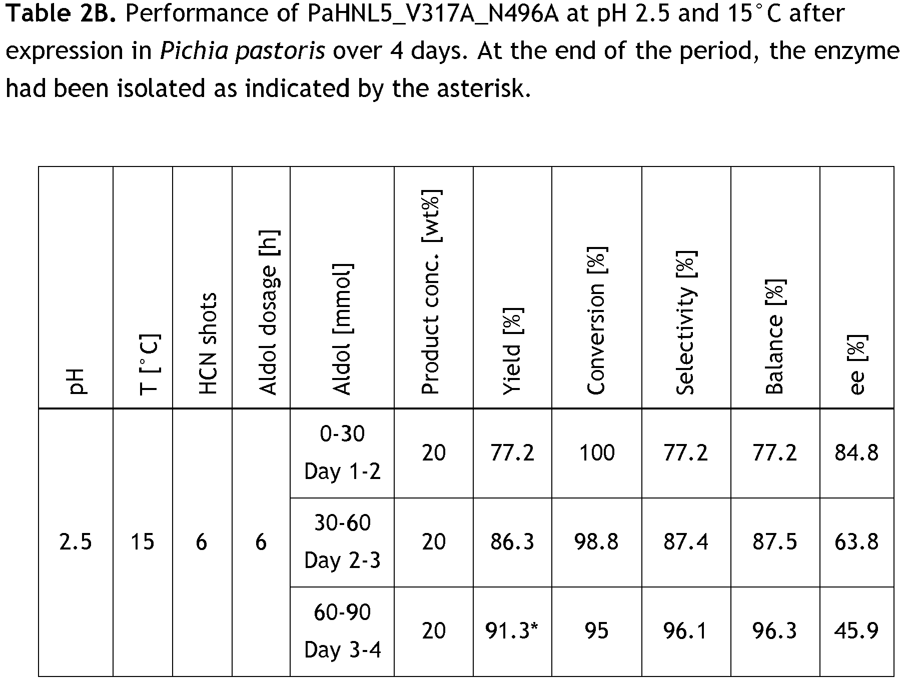

### Example 5: Performance of PaHNL5_V317A_N496S under high enzyme loading and fast substrate addition at 15°C and various pH

Further investigations were carried out to determine the influence of pH on stability of PaHNL5_V317A_N496S in the presence of 3-fold more enzyme than under standard reaction conditions and with 6-fold faster substrate addition rate. Three series of reactions were set up that spanned over a total of 3 days and involved 2 sequential reactions using the same enzyme batch. On Day 1, 3000 AMU (330 AMU/g aldol; 2000 AMU/g/h aldol dosage) of the mutant enzyme were charged into the reactor, then crystallized aldol was monomerized at 75°C for 2-3 h and added as a 20% aqueous solution over 6 h via a heated pump set to 80°C, while freshly prepared HCN (1.5 mol eq. relative to aldol) was added in 6 equal portions (0.25 mol eq. per portion) every hour during aldol addition. In all cases, pH was maintained at 3.0 and temperature at 15°C on Day 1. On Day 2, excess HCN was flushed away of the reactor using a nitrogen gas stream and when applicable the pH was adjusted to 2.5 or even 2.0 with sulfuric acid. The standard amount of monomerized aldol (30 mmol, 3.05 g) was added over 1 hour to the reaction mixture at a 6-fold faster rate than usual via a heated pump set to 80°C. In parallel, freshly prepared HCN (1.5 mol eq. relative to aldol) was added in one portion at the onset of aldol addition. From Day 2 onwards, product concentration in the reaction mixture was steady at 20 wt%. Results are summarized in Table 3 and Figure 3.

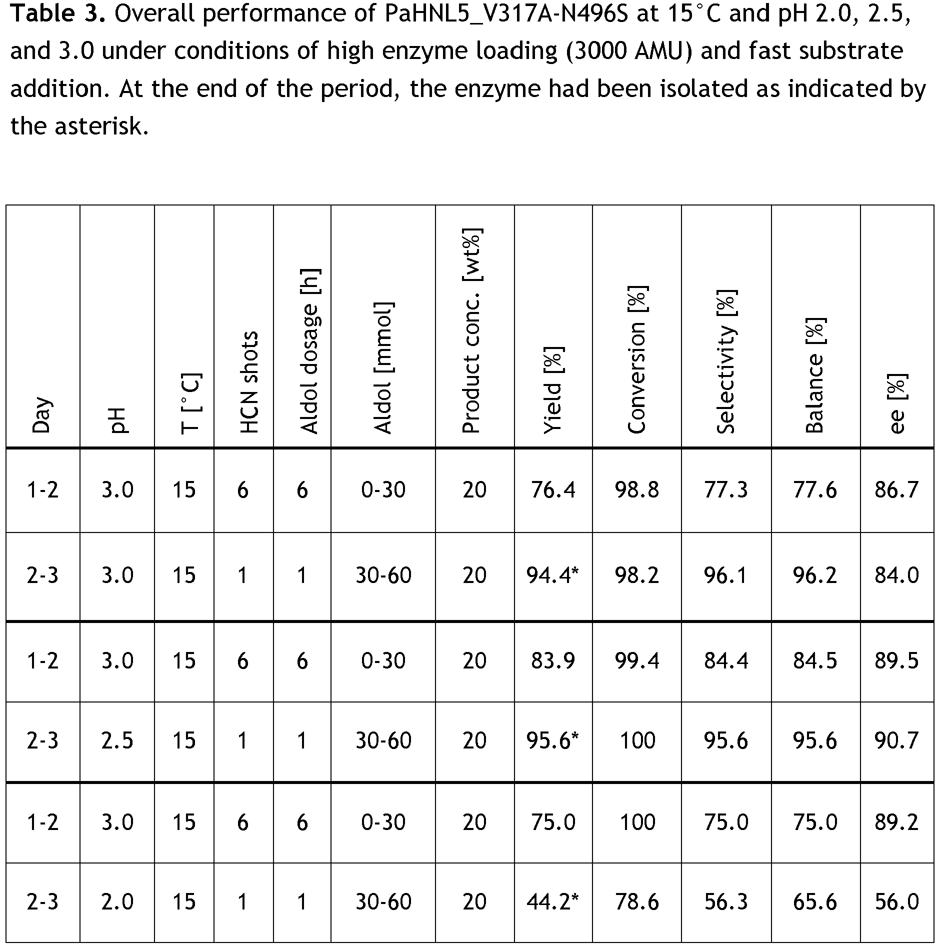

Under conditions of fast aldol addition (1 h) and high biocatalyst loading (3000 AMU), enzyme PaHNL5_V317A_N496S converts all available monomer in less than 1.5 h at pH 3.0; still the limitation remains the rate of dimer to monomer conversion. An optimum is observed at pH 2.5 where monomerization speed, enzyme stability, suppression of background reaction allow for a relatively short residence time at 15°C. At pH 3.0 the monomerization is too slow and at pH 2.0 the enzyme is totally inactive after 3-4 h. Lower pH or higher temperature favor dimer to monomer conversion; unfortunately both low pH and high temperature have a negative impact on enzyme stability.

### Example 6: Performance of PaHNL5_V317A_N496S under high enzyme loading and fast substrate addition at pH 2.5 and various temperature

Having determined an optimal pH of 2.5 for fast dimer hydrolysis to monomer and shorter possible residence times under conditions of high enzyme loading and fast substrate addition, further investigations were necessary to determine the influence of temperature on enzyme stability under those specific reaction conditions. Three series of reactions were set up that spanned over a total of 3 days and involved two sequential reactions using the same enzyme batch. On Day 1, 3000 AMU (330 AMU/g aldol; 2000 AMU/g/h aldol dosage) of enzyme PaHNL5_V317A_N496S were charged into the reactor, then crystallized aldol was monomerized at 75°C for 2-3 h and added as a 20% aqueous solution over 6 h via a heated pump set to 80°C, while freshly prepared HCN (1.5 mol eq. relative to aldol) was added in 6 equal portions (0.25 mol eq. per portion) every hour during aldol addition. In all cases, pH was maintained at 3.0 and temperature at 15°C on Day 1. Upon completion of the reaction set on Day 1, product concentration in the reaction mixture remained steady at 20 wt%. On Day 2, excess HCN was flushed away of the reactor using a nitrogen gas stream and the pH was adjusted to 2.5 with sulfuric acid. With the exception of the control reaction run at 15°C, the temperature was controlled at 20 and 25°C in the other two experiments. The standard amount of monomerized aldol (30 mmol, 3.05 g) was added over 1 hour to the reaction mixture at a 6-fold faster rate than usual via a heated pump set to 80°C. In parallel, freshly prepared HCN (1.5 mol eq. relative to aldol) was added in one portion at the onset of aldol addition. Results are summarized in Figure 4 and Table 4.

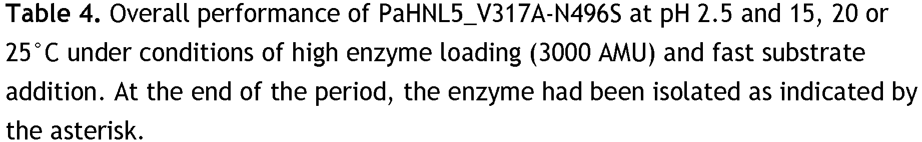

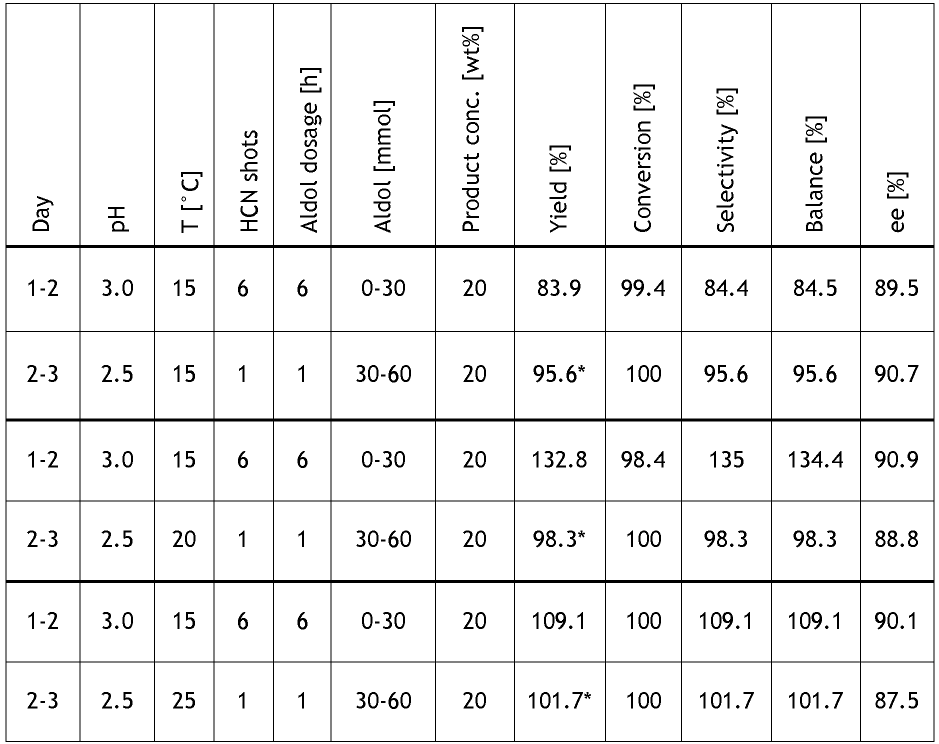

Under conditions of fast aldol addition (1 h) and high biocatalyst loading (3000 AMU), enzyme PaHNL5_V317A_N496S converts all available monomer in less than 3-4 h at pH 2.5 and 25°C (see Figure 4). Still the limitation remains the rate of dimer to monomer conversion. Higher temperatures do not lead to faster enzymatic reaction at pH 2.5 but higher temperatures do speed up the dimer hydrolysis to the active substrate monomer form. Unfortunately, increasing temperatures also speed up the background reaction. For temperatures between 15 and 25°C the enantiomeric excess remains higher than 86% (see Table 4). At 25°C, total conversion reaches about 95% already 1 h after the end of aldol addition. At 15 and 20°C, total conversion is about 90% also 1 h after the end of aldol addition.

### Example 7: Performance of PaHNL5_V317A_N496S at pH 2.5 or 3.0 and 15°C under fed-batch conditions

Investigations were carried out to determine the influence of pH on stability of enzyme PaHNL5-V317A-N496S under semi-continuous fed-batch conditions as described below:
Two series of extended reactions were set up that spanned over a total of three days and involved three sequential reactions using the same enzyme batch. In order to evaluate its impact on enzyme stability, pH was maintained at 2.5 for the first series of reactions and at 3.0 for the second series of reactions.

Temperature was controlled at 15°C throughout Day 1 and Day 2 and on Day 3 at 15°C for 8 h or until all aldol additions were completed, at which point temperature was raised to 25°C to speed up dimer hydrolysis to monomer and drive the reaction to completion in the last 12 hours of the experiment. On Day 1, 3000 AMU (330 AMU/g aldol; 2000 AMU/g/h aldol dosage) of enzyme PaHNL5_V317A_N496S were charged into the reactor under controlled pH and temperature conditions. The reaction started (t = 0) when crystallized aldol (3.05 g, monomerized at 75°C for 1 h) was added as a 20% aqueous solution over 1 h via a heated pump set to 80°C, while freshly prepared HCN (1.2 mol. eq. relative to aldol) was added in 2 unequal portions (0.3 mol. eq. at t = 0 then 0.9 mol. eq. 15 min into aldol addition) to the reaction mixture. Addition of monomerized aldol (3.05 g, 80°C, over 1 h) and HCN (1.2 mol. eq. portion wise as describe above) was repeated twice more at 2-h intervals so that a total of 9.16 g (90 mmol) of monomerized aldol was added to the reaction mixture by the end of Day 1. From Day 2 onwards, product concentration in the reaction mixture was maintained steady at 20 wt%. On Day 2, excess HCN was flushed away of the reactor using a nitrogen gas stream and the pH re-adjusted as appropriate with sulfuric acid. The standard amount of monomerized aldol (3 x 30 mmol, 3 x 3.05 g) was slowly added (over 3 x 1 h) to the reaction mixture via a heated pump set to 80°C. In parallel, freshly prepared HCN (3 x 1.2 mol. eq. relative to aldol) was added portion wise as described above. On Day 3, excess HCN was again flushed away of the reactor using a nitrogen gas stream and the pH re-adjusted as appropriate with sulfuric acid. Again, monomerized aldol (3 x 30 mmol, 3 x 3.05 g, over 3 x 1 h) was added to the reaction mixture via a heated pump set to 80°C and freshly prepared HCN (3 x 1.2 mol. eq. relative to aldol) was added in portions as described above. Temperature was raised from 15 to 25°C for the last 12 h of the reaction. Results are summarized in Table 5 and Figure 5.

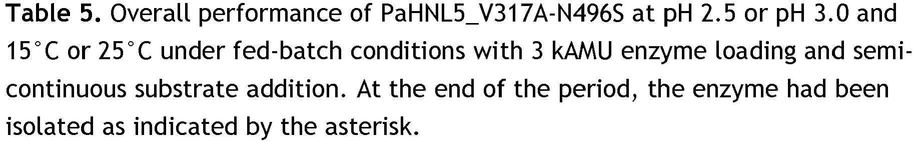

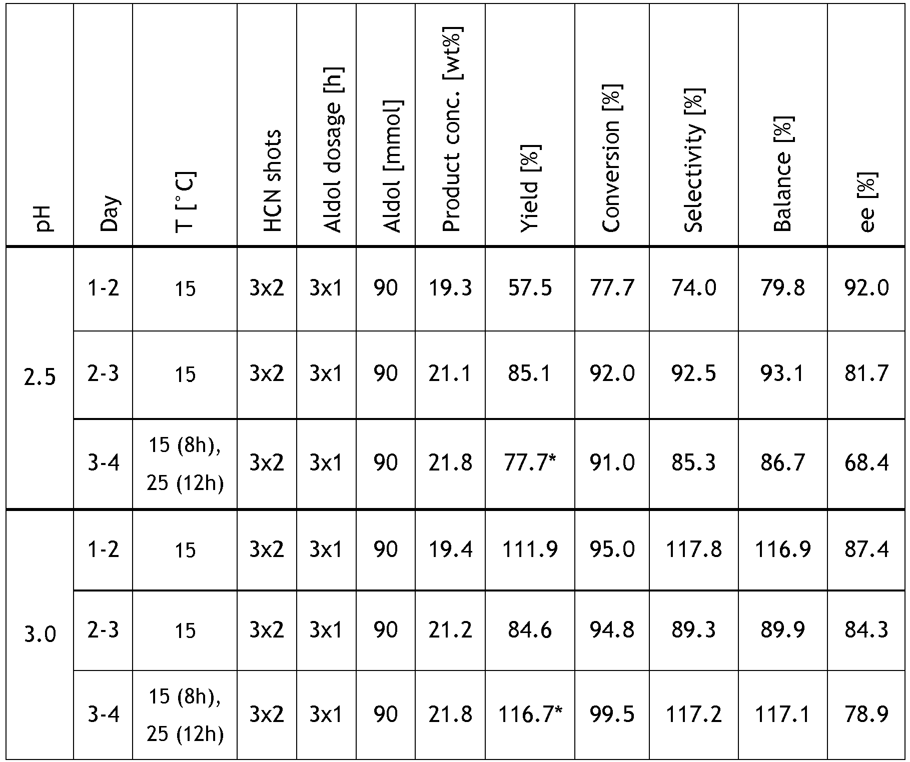

In both experiment series, a lot of solid precipitation was observed throughout the 3-day experiments, which may be either due to dimer insolubility under the reaction conditions (low pH and temperature) or to salt formation.

When the reaction series was run at pH 2.5, a steady decrease in enantiomeric excess was recorded from the onset of Day 2 until the end of the experiment on Day 3. The portion of product that stemmed from the background reaction clearly increased while the fraction produced via enzyme catalysis steadily decreased over time, pointing towards destruction of enzyme activity under the reaction conditions. With regards to overall results, the reaction did not reach completion even after 3 days. Only about 88% aldol was converted into (RS)-cyanohydrin, with the enzymatic reaction accounting for 68% of the formed product and the background reaction for 32% of product.

Under conditions where pH was controlled at 3.0, the product fraction responsible for the non-enzymatic background reaction did not increase as drastically as under conditions where pH was controlled at 2.5. The enzyme is clearly more stable at pH 3.0 than it is at pH 2.5. Moreover, the biocatalytic transformation that produced (R)-cyanohydrin remained the major aldol hydrocyanation pathway throughout the 3 days. With regards to overall results, the reaction reached completion after 3 days. No residual dimer or monomer could be detected after 3-day reaction time. The biocatalytic manifold accounted for over 79% of the formed product while the background reaction was responsible for 21% of the formed (RS)-cyanohydrin.

### Example 8: Performance of PaHNL5_V317A_N496S at pH 3.0 and 15°C using excess enzyme

Further investigations were carried out to determine whether the competing background reaction could be further suppressed at pH 3.0 by increasing the amount of enzyme PaHNL5_V317A_N496S under semi-continuous fed-batch conditions as described above.

Again, two series of experiments were set up that spanned over a total of three days, each series involving three sequential reactions using the same enzyme batch. For both reaction series, pH was maintained at 3.0 throughout the experiment. Temperature was controlled at 15°C throughout Day 1 and Day 2 and for 8 h on Day 3 until all aldol additions were completed, at which point temperature was raised to 25°C to speed up dimer hydrolysis to monomer in the last 12 hours of the experiment. For the reaction series with increased enzyme loading, 9 kAMU (330 AMU/g aldol; 2940 AMU/g/h aldol dosage) of enzyme PaHNL5_V317A_N496S were charged into the reactor under controlled pH and temperature conditions on Day 1. This reaction series was compared to a reaction wherein 3 kAMU (110 AMU/g aldol; 980 AMU/g/h aldol dosage) of enzyme PaHNL5_V317A_N496S were charged into the reactor under controlled pH and temperature conditions on Day 1. The experiments started (t=0) when crystallized aldol (3.05 g, monomerized at 75°C for 1 h) was added as a 20% aqueous solution over 1 h via a heated pump set to 80°C, while freshly prepared HCN (1.2 mol eq. relative to aldol) was added in 2 unequal portions (0.3 mol eq. at t=0 then 0.9 mo. eq. 15 min into aldol addition) to the reaction mixture. Addition of monomerized aldol (3.05 g, 80 C, over 1 h) and HCN (1.2 mol eq. portion wise as describe above) was repeated twice more at 2-h intervals so that a total of 9.16 g (90 mmol) of monomerized aldol is added to the reaction mixture by the end of Day 1. From Day 2 onwards, product concentration in the reaction mixtures was maintained steadily at 20 wt%. On Day 2, excess HCN was flushed away of the reactor using a nitrogen gas stream and the pH re-adjusted as appropriate with sulfuric acid. The standard amount of monomerized aldol (3 x 30 mmol, 3 x 3.05 g) was slowly added (over 3 x 1 h) to the reaction mixture via a heated pump set to 80°C. In parallel, freshly prepared HCN (3 x 1.2 mol eq. relative to aldol) was added portion wise as described above. On Day 3, excess HCN was again flushed away of the reactor using a nitrogen gas stream and the pH re-adjusted as appropriate with sulfuric acid. Again, monomerized aldol (3 x 30 mmol, 3 x 3.05 g, over 3 x 1 h) was added to the reaction mixture via a heated pump set to 80°C and freshly prepared HCN (3 x 1.2 mol eq. relative to aldol) was added portion wise as described above. Temperature was increased from 15 to 25°C for the last 12 h of the reaction. Results are summarized in Table 6 and Figure 6.

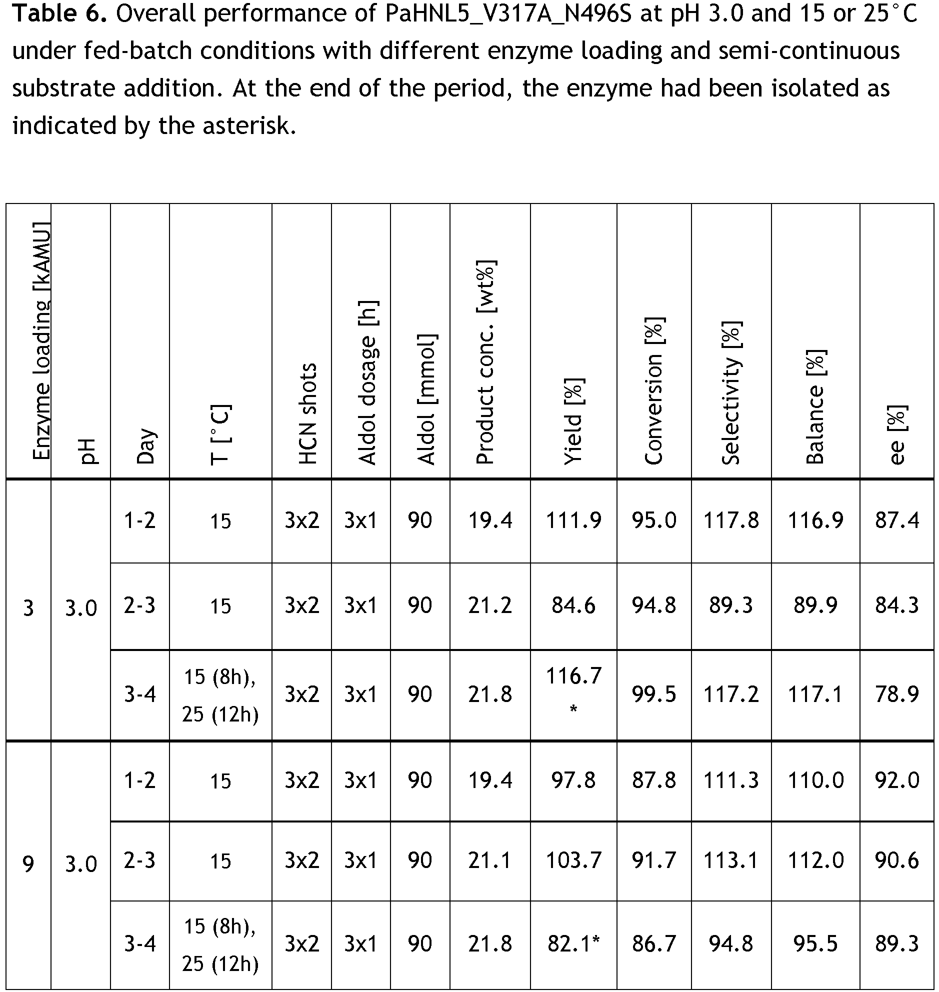

As stated previously, a lot of solid precipitation was observed in both fed-batch experiments. Interestingly, when using excess enzyme the enantiomeric excess of the product remained stable throughout the three days at around 90%, which is finally well within the process boundaries. The background reaction did not progress much between days 1, 2, or 3, pointing that enzyme PaHNL5_V317A_N496S is stable under such reaction conditions. Surprisingly, the reaction did not reach completion when using excess 9 kAMU enzyme as compared to 3 kAMU enzyme. The portion of product stemming from enzyme-catalyzed hydrocyanation of the aldol monomer was roughly the same when using 3 or 9 kAMU. In contrast, the background reaction is much more under control under conditions using excess enzyme. As a result, 89% of formed product originated from enzyme catalysis under high enzyme loading (9 kAMU) versus 79% under 3 kAMU enzyme loading. Conversion of substrate did not reach completion under conditions of excess enzyme with only 87% of aldol converted to (RS)-cyanohydrin and 83% aldol converted to (R)-cyanohydrin. Still an enantiomeric of 89% was recorded. A biocatalytic process using excess enzyme could be further optimized, e.g. by improving enzyme expression and optimizing enzyme recovery.

### Example 9: Performance of PaHNL5_V317A_N496S at 15°C with change of pH from 2.5 during substrate addition to 3.0

Further investigations were carried out to determine whether the time-dependent destruction of enzyme activity at pH 2.5 could be counterbalanced by increasing the pH to 3.0 during a limited time period thus extending the biocatalytic lifetime of a standard amount of enzyme PaHNL5_V317A_N496S under semi-continuous fed-batch conditions as described above. By maintaining pH at 2.5 during the time required for aldol addition (∼8 h), the competing background reaction is suppressed and therefore all aldol monomer present in the reaction mixture should be smoothly converted into the desired (R)-cyanohydrin up to the point when remaining substrate is solely in the unreactive dimer form. Given that hydrolysis of the dimer to the monomer is almost equally limiting at pH 2.5 and pH 3.0, raising pH to 3.0 for the time needed to convert residual dimer into monomer and reach total conversion (-12 h) could be a viable compromise between increased competition from the racemic background reaction at pH 3.0 versus pH 2.5 and increased enzyme lifespan at pH 3.0 versus pH 2.5.

Again, two series of experiments were set up that spanned over a total of three days, each series involving three sequential reactions using the same enzyme batch. For both reaction series, temperature was controlled at 15°C throughout Day 1 and Day 2 and for 8 h on Day 3 until all aldol additions were completed, at which point temperature was raised to 25°C to speed up dimer hydrolysis to monomer in the last 12 hours of the experiment. The pH was maintained at 2.5 for the first series of control reactions and cycled between pH 2.5 (for 8 h during substrate addition) and 3.0 (for 12 h or until complete conversion) for the second series of reaction. On Day 1, 3 kAMU (110 AMU/g aldol; 980 AMU/g/h aldol dosage) of enzyme PaHNL5_V317A_N496S were charged into the reactor under controlled pH and temperature conditions. The reaction started (t=0) when crystallized aldol (3.05 g, monomerized at 75°C for 1 h) was added as a 20% aqueous solution over 1 h via a heated pump set to 80°C, while freshly prepared HCN (1.2 mol eq. relative to aldol) was added in 2 unequal portions (0.3 mol eq. at t=0 then 0.9 mol eq. 15 min into aldol addition) to the reaction mixture. Addition of monomerized aldol (3.05 g, 80°C, over 1 h) and HCN (1.2 mol eq. portion wise as described above) was repeated twice more at 2-h intervals so that a total of 9.16 g (90 mmol) of monomerized aldol was added to the reaction mixture by the end of Day 1. At t=8 h, pH was either left unchanged or increased from 2.5 to 3.0. From Day 2 onwards, product concentration in the reaction mixtures was maintained steadily at 20 wt%. On Day 2, excess HCN was flushed away of the reactor using a nitrogen gas stream and the pH was re-adjusted as appropriate to pH 2.5 with sulfuric acid. The standard amount of monomerized aldol (3 x 30 mmol, 3 x 3.05 g) was slowly added (over 3 x 1 h) to the reaction mixture via a heated pump set to 80°C. In parallel, freshly prepared HCN (3 x 1.2 mo. eq. relative to aldol) was added in portion wise as described above. On Day 3, excess HCN is again flushed away of the reactor using a nitrogen gas stream and the pH re-adjusted as appropriate to pH 2.5 with sulfuric acid. Again, monomerized aldol (3 x 30 mmol, 3 x 3.05 g, over 3 x 1 h) was added to the reaction mixture via a heated pump set to 80°C and freshly prepared HCN (3 x 1.2 mol eq. relative to aldol) was added portion wise as described above. Temperature was raised from 15 to 25°C for the last 12 h of the reaction. Results are summarized in Table 7 and Figure 7.

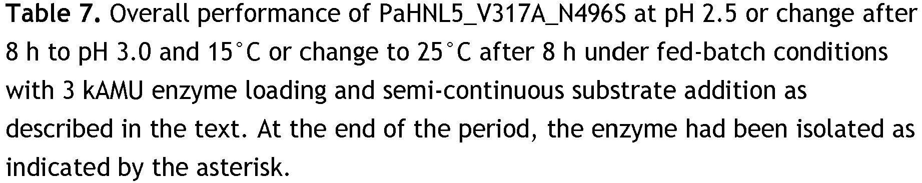

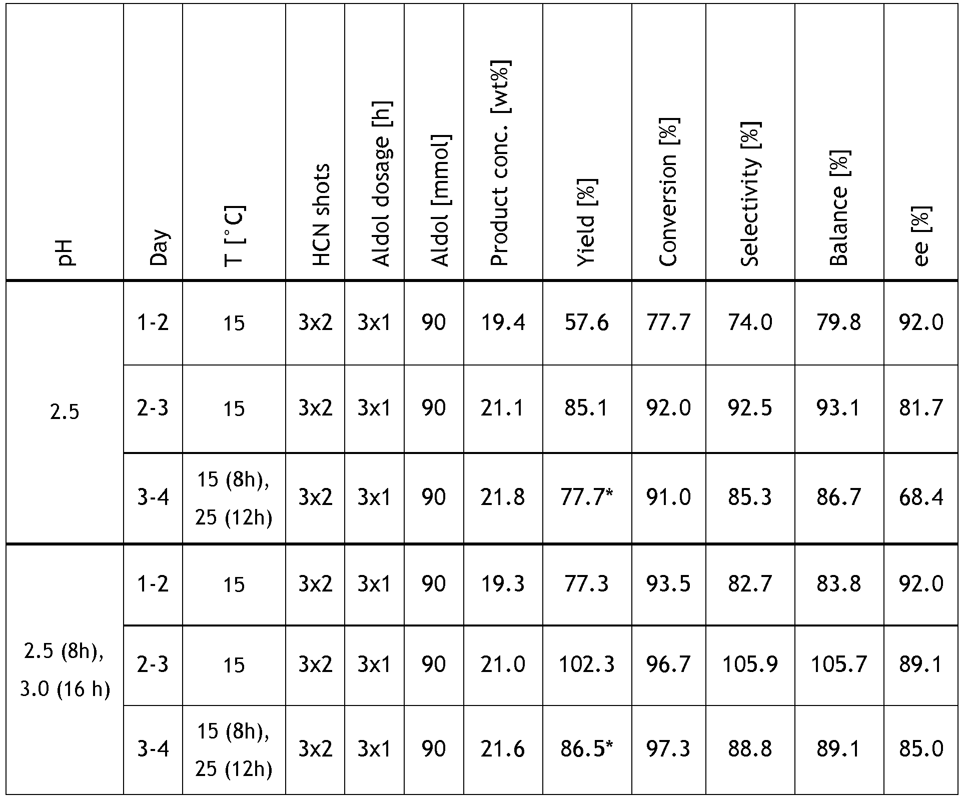

As stated in the previous section, a lot of solid precipitation was again observed in both fed-batch experiments. Interestingly, by cycling the pH between 2.5 (for 8 h during aldol addition) and 3.0 (overnight to preserve enzyme stability and allow for dimer hydrolysis to monomer) the enantiomeric excess of the product was relatively preserved throughout the three days ranging from 92% at the end of Day 1 to 85% at the end of Day 3, which is quite close to the process boundaries. This is in sharp contrast to running the experiment at a fixed pH of 2.5 as the enantiomeric excess then decreased to 68% by the end of Day 3 as enzyme activity was destroyed by the harsh reaction conditions. Again under conditions of pH cycling the reaction does not reach completion. However, all dimer was consumed and a minor amount of monomer (2-3%) remained unreacted. Indeed, conversion was greatly improved when cycling pH between 2.5 and 3.0, reaching 97% total conversion and 90% yield on (R)-cyanohydrin, as compared to controlling the pH at 2.5 throughout the experiment, which resulted in 88% and 74% conversion and yield, respectively. When cycling pH between 2.5 and 3.0, the portion of product stemming from enzyme-catalyzed hydrocyanation of the aldol monomer improved by 38% and the background reaction did not progress much between days 1, 2, or 3, pointing that enzyme PaHNL5_V317A_N496S was stable under such reaction conditions.

## Claims

1. A recombinant R-hydroxynitrile lyase (R-HNL5) from Rosaceae family member which is stable at pH 2.5 or lower for at least 1 hour and comprising the amino acid substitution V317A and amino acid substitution N496S on a position corresponding to position V317 and N496 in the mature PaHNL5 from Prunus amygdalus according to SEQ ID NO:1.

2. A recombinant R-HNL5 according to claim 1, wherein the Rosaceae family member is selected from the group consisting of Prunus amygdalus, Prunus seotina, Prunus avium, Prunus laurocerasus, Prunus lyonii, Prunus armaniaca, Prunus persica, Prunus domestica, Prunus mume, Malus communis, Malus pumila, and Cydonia oblonga.

3. The use of a recombinant R-HNL5 according to any one of the preceding claims for preparing enantiomerically pure cyanohydrins from aliphatic, aromatic or heteroaromatic aldehydes or ketones.

4. The use according to claim 3 for preparing an enantiomeric excess of at least 83% after a catalytic reaction at pH 2.5 for 1 day.

5. The use according to claim 3 or 4 for preparing R-pantolactone.

6. A process for the production of enantiomerically pure cyanohydrins comprising converting a substrate selected from aliphatic, aromatic or heteroaromatic aldehydes or ketones to the corresponding cyanohydrins by a recombinant R-HNL5 according to claim 1 or 2 in the presence of a cyanide group donor, the conversion being performed at a pH 2.5 and at temperature between 15 to 25°C.

7. A process according to claim 6, wherein the temperature at the start of the reaction is 15°C with a change to 25°C after 75% of the reaction time and wherein the pH is cycling between 2.5 and 3.0.

8. A process according to claim 6 or 7 with a conversion to (RS)-cyanohydrin of the substrate of 100% comprising cycling of pH between 3.0 and 2.5 at 15°C.

9. A process according to any one of claims 6 to 8, wherein the substrate is added continuously or semi-continuously.

10. A process according to claim 9, wherein the substrate is added over a period of more than 1 hour.

11. A process according to claim 10, wherein the substrate is added within 6 hours.

12. A process according to any one of claims 6 to 8, wherein the substrate is added within 1 hour and the amount of recombinant R-HNL is above 1 kAMU/g/h substrate dosage.

## Patentansprüche

1. Rekombinante R-Hydroxynitrillyase (R-HNL5) aus einem Mitglied der Familie Rosaceae, die bei pH 2,5 oder darunter für mindestens 1 Stunde stabil ist und die Aminosäuresubstitution V317A und die Aminosäuresubstitution N496S an einer Position umfasst, die Position V317 und N496 in der reifen PaHNL5 aus Prunus amygdalus gemäß SEQ ID NO: 1 entspricht.

2. Rekombinante R-HNL5 nach Anspruch 1, wobei das Mitglied der Familie Rosaceae aus der Gruppe ausgewählt ist, bestehend aus Prunus amygdalus, Prunus seotina, Prunus avium, Prunus laurocerasus, Prunus lyonii, Prunus armaniaca, Prunus persica, Prunus domestica, Prunus mume, Malus communis, Malus pumila und Cydonia oblonga.

3. Verwendung einer rekombinanten R-HNL5 nach einem der vorhergehenden Ansprüche zur Herstellung enantiomerenreiner Cyanhydrine aus aliphatischen, aromatischen oder heteroaromatischen Aldehyden oder Ketonen.

4. Verwendung nach Anspruch 3 zur Herstellung eines Enantiomerenüberschusses von mindestens 83% nach einer katalytischen Reaktion bei pH 2,5 für 1 Tag.

5. Verwendung nach Anspruch 3 oder 4 zur Herstellung von R-Pantolacton.

6. Verfahren zur Herstellung enantiomerenreiner Cyanhydrine, umfassend das Umwandeln eines aus aliphatischen, aromatischen oder heteroaromatischen Aldehyden oder Ketonen ausgewählten Substrats in die entsprechenden Cyanhydrine durch eine rekombinante R-HNL5 nach Anspruch 1 oder 2 in Gegenwart eines Cyanidgruppendonors, wobei die Umwandlung bei einem pH-Wert von 2,5 und einer Temperatur zwischen 15 bis 25°C durchgeführt wird.

7. Verfahren nach Anspruch 6, wobei die Temperatur zu Beginn der Reaktion 15°C beträgt, mit einer Veränderung auf 25°C nach 75% der Reaktionszeit, und wobei der pH-Wert zwischen 2,5 und 3,0 schwankt.

8. Verfahren nach Anspruch 6 oder 7 mit einer Umwandlung des Substrats in (RS)-Cyanhydrin von 100%, das das Schwanken des pH-Werts zwischen 3,0 und 2,5 bei 15°C umfasst.

9. Verfahren nach einem der Ansprüche 6 bis 8, wobei das Substrat kontinuierlich oder semikontinuierlich zugegeben wird.

10. Verfahren nach Anspruch 9, wobei das Substrat über einen Zeitraum von mehr als 1 Stunde zugegeben wird.

11. Verfahren nach Anspruch 10, wobei das Substrat innerhalb von 6 Stunden zugegeben wird.

12. Verfahren nach einem der Ansprüche 6 bis 8, wobei das Substrat innerhalb von 1 Stunde zugegeben wird und die Menge an rekombinanter R-HNL größer als 1 kAMU/g/Std. Substratdosierung ist.

## Revendications

1. R-Hydroxynitrile lyase recombinante (R-HNL5) d'un membre de la famille des Rosaceae qui est stable à pH 2,5 ou moins pendant au moins 1 heure et comprenant la substitution d'acide aminé V317A et la substitution d'acide aminé N496S à une position correspondant à la position V317 et N496 dans la PaHNL5 mature de Prunus amygdalus selon SEQ ID NO: 1.

2. R-HNL5 recombinante selon la revendication 1, le membre de la famille des Rosaceae étant choisi dans le groupe constitué de Prunus amygdalus, Prunus seotina, Prunus avium, Prunus laurocerasus, Prunus lyonii, Prunus armaniaca, Prunus persica, Prunus domestica, Prunus mume, Malus communis, Malus pumila et Cydonia oblonga.

3. Utilisation d'une R-HNL5 recombinante selon l'une quelconque des revendications précédentes pour préparer des cyanohydrines énantiomériquement pures à partir d'aldéhydes ou cétones aliphatiques, aromatiques ou hétéroaromatiques.

4. Utilisation selon la revendication 3 pour préparer un excès énantiomérique d'au moins 83 % après une réaction catalytique à pH 2,5 pendant 1 jour.

5. Utilisation selon la revendication 3 ou 4 pour préparer la R-pantolactone.

6. Procédé de production de cyanohydrines énantiomériquement pures comprenant la conversion d'un substrat choisi parmi des aldéhydes ou cétones aliphatiques, aromatiques ou hétéroaromatiques en cyanohydrines correspondantes par une R-HNL5 recombinante selon la revendication 1 ou 2 en présence d'un donneur de groupe cyanure, la conversion étant effectuée à un pH de 2,5 et à une température comprise entre 15 et 25 °C.

7. Procédé selon la revendication 6, dans lequel la température au début de la réaction est de 15 °C avec un changement à 25 °C après 75 % du temps de réaction et dans lequel le pH est soumis à un cyclage entre 2,5 et 3,0.

8. Procédé selon la revendication 6 ou 7 avec une conversion en (RS)-cyanohydrine du substrat de 100 % comprenant le cyclage de pH entre 3,0 et 2,5 à 15 °C.

9. Procédé selon l'une quelconque des revendications 6 à 8, dans lequel le substrat est ajouté de façon continue ou semi-continue.

10. Procédé selon la revendication 9, dans lequel le substrat est ajouté sur une période de plus de 1 heure.

11. Procédé selon la revendication 10, dans lequel le substrat est ajouté dans un délai de 6 heures.

12. Procédé selon l'une quelconque des revendications 6 à 8, dans lequel le substrat est ajouté dans un délai de 1 heure et la quantité de R-HNL recombinante est supérieure à une dose de substrat de 1 kAMU/g/h.


### SEQUENCE LISTING

<110> DSM IP Assets B.V.
<120> HNL mutants at low pH
<130> Case 29306
<160> 2
<170> PatentIn version 3.5
<210> 1
   <211> 532
   <212> PRT
   <213> Prunus amygdalus
<400> 1
   Leu Ala Asn Thr Ser Ala His Asp Phe Ser Tyr Leu Lys Phe Val Tyr
   1               5                   10                  15
   Asn Ala Thr Asp Thr Ser Ser Glu Gly Ser Tyr Asp Tyr Ile Val Ile
               20                  25                  30
   Gly Gly Gly Thr Ser Gly Cys Pro Leu Ala Ala Thr Leu Ser Glu Lys
           35                  40                  45
   Tyr Lys Val Leu Leu Leu Glu Arg Gly Thr Ile Ala Thr Glu Tyr Pro
       50                  55                  60
   Asn Thr Leu Thr Ala Asp Gly Phe Ala Tyr Asn Leu Gln Gln Gln Asp
   65                  70                  75                  80
   Asp Gly Lys Thr Pro Val Glu Arg Phe Val Ser Glu Asp Gly Ile Asp
                   85                  90                  95
   Asn Val Arg Ala Arg Ile Leu Gly Gly Thr Thr Ile Ile Asn Ala Gly
               100                 105                 110
   Val Tyr Ala Arg Ala Asn Ile Ser Phe Tyr Ser Gln Thr Gly Ile Glu
           115                 120                 125
   Trp Asp Leu Asp Leu Val Asn Lys Thr Tyr Glu Trp Val Glu Asp Ala
       130                 135                 140
   Ile Val Val Lys Pro Asn Asn Gln Ser Trp Gln Ser Val Ile Gly Glu
   145                 150                 155                 160
   Gly Phe Leu Glu Ala Gly Ile Leu Pro Asp Asn Gly Phe Ser Leu Asp
                   165                 170                 175
   His Glu Ala Gly Thr Arg Leu Thr Gly Ser Thr Phe Asp Asn Asn Gly
               180                 185                 190
   Thr Arg His Ala Ala Asp Glu Leu Leu Asn Lys Gly Asp Pro Asn Asn
           195                 200                 205
   Leu Leu Val Ala Val Gln Ala Ser Val Glu Lys Ile Leu Phe Ser Ser
       210                 215                 220
   Asn Thr Ser Asn Leu Ser Ala Ile Gly Val Ile Tyr Thr Asp Ser Asp
   225                 230                 235                 240
   Gly Asn Ser His Gln Ala Phe Val Arg Gly Asn Gly Glu Val Ile Val
                   245                 250                 255
   Ser Ala Gly Thr Ile Gly Thr Pro Gln Leu Leu Leu Leu Ser Gly Val
               260                 265                 270
   Gly Pro Glu Ser Tyr Leu Ser Ser Leu Asn Ile Thr Val Val Gln Pro
           275                 280                 285
   Asn Pro Tyr Val Gly Gln Phe Val Tyr Asp Asn Pro Arg Asn Phe Ile
       290                 295                 300
   Asn Ile Leu Pro Pro Asn Pro Ile Glu Ala Ser Val Val Thr Val Leu
   305                 310                 315                 320
   Gly Ile Arg Ser Asp Tyr Tyr Gln Val Ser Leu Ser Ser Leu Pro Phe
                   325                 330                 335
   Ser Thr Pro Pro Phe Ser Leu Phe Pro Thr Thr Ser Tyr Pro Leu Pro
               340                 345                 350
   Asn Ser Thr Phe Ala His Ile Val Ser Gln Val Pro Gly Pro Leu Ser
           355                 360                 365
   His Gly Ser Val Thr Leu Asn Ser Ser Ser Asp Val Arg Ile Ala Pro
       370                 375                 380
   Asn Ile Lys Phe Asn Tyr Tyr Ser Asn Ser Thr Asp Leu Ala Asn Cys
   385                 390                 395                 400
   Val Ser Gly Met Lys Lys Leu Gly Asp Leu Leu Arg Thr Lys Ala Leu
                   405                 410                 415
   Glu Pro Tyr Lys Ala Arg Asp Val Leu Gly Ile Asp Gly Phe Asn Tyr
               420                 425                 430
   Leu Gly Val Pro Leu Pro Glu Asn Gln Thr Asp Asp Ala Ser Phe Glu
   435 440 445
   Thr Phe Cys Leu Asp Asn Val Ala Ser Tyr Trp His Tyr His Gly Gly
   450 455 460
   Ser Leu Val Gly Lys Val Leu Asp Asp Ser Phe Arg Val Met Gly Ile
   465 470 475 480
   Lys Ala Leu Arg Val Val Asp Ala Ser Thr Phe Pro Tyr Glu Pro Asn
   485 490 495
   Ser His Pro Gln Gly Phe Tyr Leu Met Leu Gly Arg Tyr Val Gly Leu
   500 505 510
   Gln Ile Leu Gln Glu Arg Ser Ile Arg Leu Glu Ala Ile His Asn Ile
   515 520 525
   Gln Glu Ser Met
   530
<210> 2
   <211> 532
   <212> PRT
   <213> Artificial sequence
<220>
   <223> modified PaHNL5
<400> 2
   Leu Ala Asn Thr Ser Ala His Asp Phe Ser Tyr Leu Lys Phe Val Tyr
   1 5 10 15
   Asn Ala Thr Asp Thr Ser Ser Glu Gly Ser Tyr Asp Tyr Ile Val Ile
   20 25 30
   Gly Gly Gly Thr Ser Gly Cys Pro Leu Ala Ala Thr Leu Ser Glu Lys
   35 40 45
   Tyr Lys Val Leu Leu Leu Glu Arg Gly Thr Ile Ala Thr Glu Tyr Pro
   50 55 60
   Asn Thr Leu Thr Ala Asp Gly Phe Ala Tyr Asn Leu Gln Gln Gln Asp
   65 70 75 80
   Asp Gly Lys Thr Pro Val Glu Arg Phe Val Ser Glu Asp Gly Ile Asp
   85 90 95
   Asn Val Arg Ala Arg Ile Leu Gly Gly Thr Thr Ile Ile Asn Ala Gly
               100                 105                 110
   Val Tyr Ala Arg Ala Asn Ile Ser Phe Tyr Ser Gln Thr Gly Ile Glu
           115                 120                 125
   Trp Asp Leu Asp Leu Val Asn Lys Thr Tyr Glu Trp Val Glu Asp Ala
       130                 135                 140
   Ile Val Val Lys Pro Asn Asn Gln Ser Trp Gln Ser Val Ile Gly Glu
   145                 150                 155                 160
   Gly Phe Leu Glu Ala Gly Ile Leu Pro Asp Asn Gly Phe Ser Leu Asp
                   165                 170                 175
   His Glu Ala Gly Thr Arg Leu Thr Gly Ser Thr Phe Asp Asn Asn Gly
               180                 185                 190
   Thr Arg His Ala Ala Asp Glu Leu Leu Asn Lys Gly Asp Pro Asn Asn
           195                 200                 205
   Leu Leu Val Ala Val Gln Ala Ser Val Glu Lys Ile Leu Phe Ser Ser
       210                 215                 220
   Asn Thr Ser Asn Leu Ser Ala Ile Gly Val Ile Tyr Thr Asp Ser Asp
   225                 230                 235                 240
   Gly Asn Ser His Gln Ala Phe Val Arg Gly Asn Gly Glu Val Ile Val
                   245                 250                 255
   Ser Ala Gly Thr Ile Gly Thr Pro Gln Leu Leu Leu Leu Ser Gly Val
               260                 265                 270
   Gly Pro Glu Ser Tyr Leu Ser Ser Leu Asn Ile Thr Val Val Gln Pro
           275                 280                 285
   Asn Pro Tyr Val Gly Gln Phe Val Tyr Asp Asn Pro Arg Asn Phe Ile
       290                 295                 300
   Asn Ile Leu Pro Pro Asn Pro Ile Glu Ala Ser Val Ala Thr Val Leu
   305                 310                 315                 320
   Gly Ile Arg Ser Asp Tyr Tyr Gln Val Ser Leu Ser Ser Leu Pro Phe
                   325                 330                 335
   Ser Thr Pro Pro Phe Ser Leu Phe Pro Thr Thr Ser Tyr Pro Leu Pro
               340                 345                 350
   Asn Ser Thr Phe Ala His Ile Val Ser Gln Val Pro Gly Pro Leu Ser
           355                 360                 365
   His Gly Ser Val Thr Leu Asn Ser Ser Ser Asp Val Arg Ile Ala Pro
       370                 375                 380
   Asn Ile Lys Phe Asn Tyr Tyr Ser Asn Ser Thr Asp Leu Ala Asn Cys
   385                 390                 395                 400
   Val Ser Gly Met Lys Lys Leu Gly Asp Leu Leu Arg Thr Lys Ala Leu
                   405                 410                 415
   Glu Pro Tyr Lys Ala Arg Asp Val Leu Gly Ile Asp Gly Phe Asn Tyr
               420                 425                 430
   Leu Gly Val Pro Leu Pro Glu Asn Gln Thr Asp Asp Ala Ser Phe Glu
           435                 440                 445
   Thr Phe Cys Leu Asp Asn Val Ala Ser Tyr Trp His Tyr His Gly Gly
       450                 455                 460
   Ser Leu Val Gly Lys Val Leu Asp Asp Ser Phe Arg Val Met Gly Ile
   465                 470                 475                 480
   Lys Ala Leu Arg Val Val Asp Ala Ser Thr Phe Pro Tyr Glu Pro Ser
                   485                 490                 495
   Ser His Pro Gln Gly Phe Tyr Leu Met Leu Gly Arg Tyr Val Gly Leu
               500                 505                 510
   Gln Ile Leu Gln Glu Arg Ser Ile Arg Leu Glu Ala Ile His Asn Ile
           515                 520                 525
   Gln Glu Ser Met
       530